# EUROPÄISCHE PATENTANMELDUNG

(11) **EP 4 324 551 A1**
(43) Veröffentlichungstag der Anmeldung: **21.02.2024**
(21) Anmeldenummer: 23191002.7
(22) Anmeldetag: 11.08.2023
(51) Int. Cl.: B01J 13/04, B01J 13/14, B01J 13/20, C08L 89/06

(54) **VERFAHREN UND VORRICHTUNG ZUR HERSTELLUNG VON HOHLKUGELN AUS KOLLAGEN UND KOLLAGENDERIVATEN UND HOHLKUGELN AUS KOLLAGEN UND KOLLAGENDERIVATEN**

(30) Priorität: 15.08.2022 DE 102022120500
(71) Anmelder: FILK Freiberg Institute gGmbH, 09599 Freiberg Sachsen (DE)
(72) Erfinder: PRADE, Ina, 09661 Hainichen (DE); MEYER, Michael, 01159 Dresden (DE); KRUMBIEGEL, Claudia, 09629 Dittmannsdorf (DE)
(74) Vertreter: Rauschenbach, Marion

(57) **Zusammenfassung**

Die Erfindung bezieht sich auf die Gebiete der Medizin- und Labortechnik sowie der Zellbiologie und betrifft ein Verfahren und eine Vorrichtung zur Herstellung von Hohlkugeln aus Kollagen und Kollagenderivaten sowie derart hergestellte Hohlkugeln. Aufgabe der Erfindung ist, eine kostengünstige und reproduzierbare Vorrichtung und ein Verfahren zur Herstellung von Hohlkugeln als in vitro-Modelle bereitzustellen und eine realitätsnahe Nachbildung von humanen Zellen und Lungenbläschen in vitro zu möglichen.

Erfindungsgemäß wird ein Verfahren und eine Vorrichtung zur Herstellung von Hohlkugeln bereitgestellt, die eine Wandung aus Kollagen und mindestens einem Kollagenderivat aufweisen, wobei der Hohlraum mit mindestens einem Gas gefüllt ist. Es werden hierfür in ein Behältnis mindestens eine Suspension oder Lösung aus Kollagen und mindestens einem Kollagenderivat, mindestens eine Separationsflüssigkeit und mindestens eine Vernetzungsflüssigkeit angeordnet, nachfolgend über eine Gaszuführeinrichtung eine Gasmischung eingeleitet, wodurch mit Gas gefüllte Hohlkugeln mit einer Kollagen-Kollagenderivat-Wandung ausgebildet und der Separationsflüssigkeit und nachfolgend der Vernetzungsflüssigkeit zugeführt werden.

## Beschreibung

Die vorliegende Erfindung bezieht sich auf die Gebiete der Medizin- und Labortechnik sowie der Zellbiologie und betrifft ein Verfahren und eine Vorrichtung zur Herstellung von Hohlkugeln aus Kollagen und Kollagenderivaten sowie derart hergestellte Hohlkugeln aus Kollagen und mindestens einem Kollagenderivat. Die mit dem erfindungsgemäßen Verfahren und der Vorrichtung hergestellten Hohlkugeln aus Kollagen und mindestens einem Kollagenderivat können beispielsweise als in vitro-Modelle, oder für das Tissue Engineering, auf dem Gebiet der Pneumologie, als Drug Delivery- und Zellträgersystem im Rahmen medizinischer Therapien, als Anhaftungs- und Kompartimentierungssystem für Hochdichtekulturen bei der Erzeugung von Biologicals und als Kapsel für biosensorische Elemente eingesetzt werden.

Während in den vergangenen Jahrzehnten in vivo-Versuche an Tiermodellen im Mittelpunkt dieser Untersuchungen standen, wird mittlerweile der Nutzen dieser regulatorisch vorgeschriebenen Tests in Frage gestellt. Für die Verwendung alternativer Modelle spricht die Tatsache, dass sich viele physiologische und pathologische Prozesse beim Menschen signifikant vom Tier unterscheiden. Darüber hinaus werden immer stärker auch ethische Fragen im Zusammenhang mit Tierversuchen diskutiert, und es werden Ersatzmethoden gesucht.

In vitro-Modelle ermöglichen es, komplexe Phänomene des menschlichen Körpers unter vereinfachten, gut kontrollierbaren und leicht zugänglichen Bedingungen abzubilden. Sie ermöglichen die Bewertung der Wirksamkeit und Sicherheit von Medikamenten und sind damit ein bedeutendes Werkzeug bei der Entwicklung neuer Therapien. Neben der Vorhersagbarkeit der Wirkung können humane Gewebemodelle unterstützend genutzt werden, um Mechanismen der Wirkung von Arzneistoffen oder Krankheitsursachen aufzuklären. Dennoch ist bekannt, dass diese Vereinfachung auch Einschränkungen mit sich bringt. Nicht nur die verwendeten Zelltypen, sondern vor allem auch das Design und die Komplexität beeinflusst die physiologische Relevanz des Modells.

Um eine große Ähnlichkeit eines in vitro-Modells zu beispielsweise den Lungenbläschen des menschlichen Körpers zu erzielen, sind verschiedene Komponenten der Lunge physiologisch relevant. Bei der Ausgestaltung eines in vitro-Modells sind zudem ein elastisches Substrat aus EZM-Komponenten für die Anhaftung der Zellen sowie die Möglichkeit der mechanischen Stimulation, die zur Bewegung der Alveolarbarriere während der Atmung führt, zu berücksichtigen.

Um ein realitätsnahes in vitro-Modell zu erzeugen, ist es erforderlich, dass möglichst viele charakteristische Merkmale eines Gewebes nachgebildet werden. Die Erzeugung einer runden Geometrie aus Kollagen wurde in der Vergangenheit vor allem für eine Anwendung im Wirkstofftransport (Drug Delivery) untersucht. Es gibt aber auch zahlreiche Gewebe im menschlichen Körper, die sich durch eine runde Form auszeichnen, wie z. B. die Lungenbläschen, die Blase oder der Kapselsack des Auges.

Aus dem Stand der Technik sind bereits verschiedene Lösungsansätze für die Erzeugung und Herstellung von in vitro-Modellen unter Einsatz von Kollagen bekannt, die die Herstellung von entweder voll ausgefüllten Kollagen-Kügelchen, sogenannte Mikrosphären, oder von Hohlkugeln (hollow spheres) beschreiben.

Die WO 2021 141 595 A1 offenbart ein Verfahren zur Herstellung von Mikrocarriern aus Kollagen zur Anwendung in der Wundheilung. Die Mikrocarrier bestehen aus Kollagen, einer stabilisierenden Fettsäure wie Stearinsäure und einem flüssigen Kern, der mit Sauerstoff und einem heilungsfördernden Wirkstoff angereichert sein kann. Eine zusätzliche Stabilisierung wird durch die Zugabe von Phospholipiden, Mono- oder Polysacchariden, Polyole, Glycoproteine und -lipide oder Phosphoproteine erreicht. Zur Herstellung der Mikrocarrier wird eine wässrige Lösung aus den bereits genannten Komponenten in einem Behältnis vorgelegt, anschließend wird Gas (z. B. Sauerstoff) über der Flüssigphase eingebracht. Durch das Anlegen von Druck und einer Anregung, beispielsweise mittels Ultraschall oder Rühren, entstehen Bläschen an der Gas-Flüssigkeitsgrenze. Durch das Herunterkühlen des Behältnisses werden die Kügelchen in der wässrigen Phase stabilisiert. Daraus ergibt sich dann eine Sammlung unterschiedlich großer Kugeln (zwischen 0,5 und 30 µm).

Aus der WO 2014 025 312 ist die Herstellung von Gelatinemikropartikeln bekannt, die mit Zellen befüllt sind. Die Zellen werden dazu in eine Gelatinelösung gegeben und mit einem Öl gemischt. Die Emulsion wird bei niedrigen Temperaturen gerührt, so dass kleine mit Zellen gefüllte Partikel entstehen. Die Partikel werden vom Öl separiert und entweder einzeln eingesetzt oder mit Alginat ummantelt. Nach dem Gelieren des Alginates wird die Gelatine durch Wärme herausgelöst und die Zellen verbleiben in einem Alginatgerüst mit runden Hohlräumen zurück.

Die EP 1 707 260 A1 beschreibt ein Verfahren zur Herstellung von vernetzten Mikrosphären aus Kollagen. Dazu wird eine saure Kollagenlösung mit einem Emulgator wie Polyvinylalkohol versetzt und mit einer hydrophoben Flüssigkeit (Chlormethan oder Ethylacetat) unter Bedingungen kombiniert, die zur Bildung einer Wasser-in-Öl-Emulsion führen. Die Wasser-in-Öl-Emulsion wird einer gepufferten Alkohollösung zugesetzt. Dabei entsteht eine Dispersion mit einer festen Phase aus rekonstituiertem fibrillierten Kollagen. Durch Entfernung der flüssigen Phase entstehen Kollagen-Mikrokugeln, die gefriertrocknet und anschließend chemisch vernetzt werden.

WO 2019 136 453 A1 offenbart die Erzeugung eines 3D-Zellkulturmodells zur Simulation der Alveoli der Lunge. Dabei werden Mikrokügelchen auf Basis verschiedener chemischer Komponenten wie PEG, Zelladhäsion fördernde Peptidsequenzen und Vernetzer hergestellt und mit Hilfe einer mikrofluidischen Vorrichtung mit Zellen versetzt. Die Kügelchen werden zusätzlich mit Magnetpartikeln beladen, um eine Aggregation mehrerer Kügelchen unter Zellkulturbedingungen zu initiieren. Die Kügelchen bestehen aus einer Kapsel und einem Inneren. Letzteres kann durch Enzyme der Zellen verdaut werden. Dabei entstehen zellbeladene Hohlkugeln. Die Kugeln werden in eine Matrix eingebunden, die in ihrer mechanischen Festigkeit variiert werden kann. Dazu werden photosensitive Hydrogele wie methacrylierte Gelatine verwendet.

Zur Bildung von 3D-Lungenmodellen sind aus dem Stand der Technik weitere Lösungen bekannt.

Aus der WO 2018 122 219 A1 und WO 2009 048 661 A1 sind Zellkulturmodelle im flächigen Format bekannt, bei denen die Zellen mehrschichtig auf einer porösen Membran wachsen.

Weiterhin ist aus der EP 2450707B1 die Kultivierung von verschiedenen Zellen bekannt, die dem Lungengewebe zugeordnet werden, ohne dass diese eine künstliche Gerüstmatrix aufweisen.

Und auch bekannt aus der WO 2014 018 691 A1 ist die Erzeugung von 3D-Sphären aus Lungenprogenitor- und Epithelzellen. Es wird beschrieben, mit welchen Wachstumsfaktoren und Signalsubstanzen die Zellen in nicht-adhäsiven Kulturgefäßen kultiviert werden müssen, um von selbst Sphären oder Hohlkugeln zu bilden.

Die Zellen im Körper befinden sich in einer komplexen Umgebung. Neben extrazellulären Matrixproteinen sind auch mechanische Kräfte zu finden, die auf die Zellen einwirken und das Verhalten und die Reaktion der Zellen beeinflusst. Die mechanische Stimulation in vitro ist bisher nicht an 3D-Geweben mit Lumen realisiert worden.

Nachteilig im Stand der Technik ist es, dass die in vitro Modellbildung von Hohlkugeln sowie deren mechanische Simulation ungenügend ist. Und auch ist nachteilig, dass die vorgeschlagenen Verfahren mit hohen Kosten und dem Einsatz aufwendiger Labortechnik mit ungenügender Reproduzierbarkeit der Modelle und einer breiten Größenverteilung der Hohlkugeln verbunden sind, die darüber hinaus nicht einzeln, sondern als Agglomerat oder als Schaum und damit als Ansammlung von Hohlkugeln unterschiedlicher Größe vorliegen.

Nachteilig bei den bisherigen in vitro-Modellen ist auch, dass beispielsweise Emulgatoren, Tenside, Stabilisatoren oder Ähnliches angewandt werden, die zu nicht definierbaren und unerwünschten Wechselwirkungen mit Zellen führen können. Aufgabe der vorliegenden Erfindung ist, eine kostengünstige, einfache, schnelle und reproduzierbare Vorrichtung und sowie ein Verfahren zur Herstellung von einzeln handelbaren Hohlkugeln als in vitro-Modelle bereitzustellen. Weiterhin besteht die Aufgabe darin, Hohlkugeln als in vitro-Modelle zur Verfügung zu stellen, die eine verbesserte realitätsnahe Nachbildung von humanen Zellen und Lungenbläschen in vitro ermöglichen.

Die Aufgabe wird mit den technischen Merkmalen des oder der Hauptansprüche gelöst. Vorteilhafte Ausgestaltungen sind Gegenstand der Unteransprüche, wobei die Erfindung auch Kombinationen der einzelnen Unteransprüche im Sinne einer und-Verknüpfung einschließt, solange sie sich nicht gegenseitig ausschließen.

Gelöst wir die erfindungsgemäße Aufgabe mit einem Verfahren zur Herstellung von Hohlkugeln aus Kollagen und Kollagenderivaten, bei dem in ein Behältnis mindestens eine Suspension oder Lösung aus Kollagen und mindestens einem Kollagenderivat, mindestens eine gegenüber der Suspension höher-viskose und niedriger-dichte Separationsflüssigkeit und mindestens eine gegenüber der Separationsflüssigkeit niedriger-viskose und niedriger-dichte Vernetzungsflüssigkeit angeordnet werden, wobei die Separationsflüssigkeit als phasentrennende Schicht zwischen der Suspension und der Vernetzungsflüssigkeit angeordnet wird, nachfolgend über mindestens eine Gaszuführeinrichtung eine Gasmischung in die Suspension oder Lösung eingeleitet wird, wodurch aus den eingeleiteten Gasbläschen mit diesem Gas gefüllte Hohlkugeln mit einer Kollagen-Kollagenderivat-Wandung ausgebildet und der Separationsflüssigkeit zugeführt werden, nachfolgend die Hohlkugeln die Separationsflüssigkeit durchdringen und nachfolgend die Hohlkugeln der Vernetzungsflüssigkeit zugeführt werden, wobei die Hohlkugeln in der Vernetzungsflüssigkeit stabilisiert und vereinzelt werden, und nachfolgend die ausgebildeten Hohlkugeln aus der Vernetzungsflüssigkeit entfernt werden, wobei danach weitere Behandlungen der Hohlkugeln durchgeführt werden können.

Vorteilhafterweise wird das Behältnis mindestens im Bereich der Suspension oder Lösung über eine zusätzliche thermische Quelle und/oder durch Zugabe mindestens eines Additives temperiert, wobei besonders vorteilhaft als Additiv Harnstoff, Guanidinhydrochlorid und/oder andere hydrotropisch wirkende Additive zugegeben werden.

Besonders vorteilhaft werden zur Dichte- und Viskositätsregulierung wasserlösliche Oligo- oder Polymere, bevorzugt Kohlenhydrate und/oder Polysaccharide zugesetzt.

In einer vorteilhaften Ausgestaltung des Verfahrens wird eine steuerbare Gaszuführeinrichtung eingesetzt, mit der das zuführbare Gasvolumen der Gasmischung einstellbar und dadurch der Außendurchmesser der Hohlkugeln aus Kollagen und Kollagenderivaten variierbar ist.

Auch ist es vorteilhaft, wenn die Suspension oder Lösung mit einem Anteil von 1 Gew.-% bis 50 Gew.-% Kollagen und Kollagenderivat verwendet wird.

Weiterhin vorteilhaft ist es, wenn als Separationsflüssigkeit eine nicht wassermischbare Flüssigkeit eingesetzt wird.

Bei einer vorteilhaften Ausgestaltung des Verfahrens wird die Separationsflüssigkeit durch mindestens einen Reinigungsvorgang mit einem Lösemittel mindestens teilweise von der hergestellten Hohlkugel entfernt, wobei besonders vorteilhaft als Lösemittel eine alkoholische Lösung, besonders vorteilhaft Ethanol, eingesetzt wird.

Zudem ist es von Vorteil, wenn in der hergestellten Hohlkugel und/oder auf dessen Wandung innen und/oder außen mindestens eine Zellsuspension, Gewebezellen und/oder Mikroorganismen inkubiert und/oder injiziert wird.

Auch vorteilhafterweise werden die Hohlkugeln nach dem Entfernen aus der Vernetzungsflüssigkeit zur Nachvernetzung einer Nachvernetzungsflüssigkeit zugeführt, wobei besonders vorteilhaft ist, wenn in der Nachvernetzungsflüssigkeit vernetzende Enzyme, bevorzugt Transglutaminase, und/oder Formaldehyd, bifunktionelle Aldehyde, Carbodiimide und/oder Isocyanate in wässriger Lösung eingesetzt werden.

Von Vorteil ist es auch, wenn das oder die Kollagenderivate durch Acrylate, Methacrylate und/oder Vinylverbindungen modifiziert werden.

Und auch von Vorteil ist es, wenn die Vernetzung und/oder die Nachvernetzung physikalisch durch elektromagnetische oder Teilchenstrahlung erfolgt.

Die erfindungsgemäße Aufgabe wird auch mit einer Vorrichtung zur Durchführung des vorbeschriebenen Verfahrens gelöst, die ein Behältnis mit mindestens einer Gaszuführeinrichtung aufweist, wobei im Behältnis eine Suspension oder Lösung aus Kollagen und mindestens einem Kollagenderivat, eine gegenüber der Suspension höher-viskose und niedriger-dichte Separationsflüssigkeit und eine gegenüber der Separationsflüssigkeit niedriger- viskose und niedriger-dichte Vernetzungsflüssigkeit vorhanden sind, wobei die Gaszuführeinrichtung im Bereich der angeordneten Suspension realisiert ist, und wobei im Bereich der Vernetzungsflüssigkeit eine Trennvorrichtung angeordnet ist.

Vorteilhafterweise ist innerhalb und/oder außerhalb des Behältnisses mindestens im Bereich der Suspension oder Lösung eine thermische Quelle angeordnet.

Ebenfalls vorteilhaft ist es, wenn die mindestens eine Gaszuführeinrichtung im Bereich des Behälterbodens oder in der Behälterseitenwand vorgesehen ist.

Auch vorteilhafterweise sind zwei oder mehrere Gaszuführeinrichtungen vorhanden, die in unterschiedlicher Behälterhöhe die Gaszufuhr in die Suspension ermöglichen.

Zudem ist es von Vorteil, wenn die Trennvorrichtung ein trichterförmiges Bauteil, Überlaufanordnung, Abschöpfeinrichtung, Absaugvorrichtung und/oder eine pneumatische Vorrichtung ist.

Gelöst wird die erfindungsgemäß Aufgabe zudem mit einer Hohlkugel, die eine multiaxial und elastisch verformbare Wandung aus Kollagen und mindestens einem Kollagenderivat aufweist, wobei mindestens der ausgebildete Hohlraum mit mindestens einem Gas gefüllt ist.

Vorteilhaft ist es, wenn das Kollagen einen oder mehrere unterschiedliche Kollagentypen enthält.

Vorteilhafterweise ist bei der Hohlkugel fibrilliertes Kollagen und als Kollagenderivat Gelatine vorhanden.

In einer vorteilhaften Ausgestaltung der Hohlkugel enthält mindestens der Hohlraum eine Flüssigkeit, Mikroorganismen, Feststoffe und/oder Zellgewebe.

Weiterhin ist vorteilhaft, wenn die Hohlkugel einen Außendurchmesser von 0,2 mm bis ≤ 1,5 cm aufweist.

Und auch vorteilhafterweise weist die Wandung der Hohlkugel eine Schichtdicke von 5 µm bis ≤ 500 µm, bevorzugt 10 µm bis 200 µm auf.

Mit der vorliegenden Erfindung werden ein Verfahren und eine Vorrichtung zur Herstellung von Hohlkugeln aus Kollagen und mindestens einem Kollagenderivat als in vitro-Modell bereitgestellt, das kostengünstig, einfach, schnell und sicher ist sowie eine hohe Reproduzierbarkeit ermöglicht. Weiterhin werden Hohlkugeln als in vitro-Modelle bereitgestellt, die eine verbesserte realitätsnahe Nachbildung und mechanische Stimulation von humanen Zellen und Lungenbläschen mit hohem Ähnlichkeitswert zum humanen Lungenbläschen mit angepassten mechanischen und physischen Eigenschaften aufweisen.

In der nachfolgenden Beschreibung, dem Ausführungsbeispiel sowie den Patentansprüchen werden die für die Erfindung wesentlichen Sachverhalte wie folgt definiert:
Unter Kollagen wird im Rahmen der Erfindung die Familie von Strukturproteinen verstanden, die bevorzugt im Bindegewebe höherer Tiere zu finden sind, gekennzeichnet durch die sich wiederholende Aminosäureabfolge (Triplett) Glycin -X - Y, wobei X häufig Prolin und Y Hydroxyprolin sein kann. In dieser Familie von Strukturproteinen werden die einzelnen Mitglieder, die in einer Spezies wenig in ihrer Sequenz abweichen durch römische Zahlen dargestellt (I...XXVIII), wobei die mengenmäßig wesentlichen Strukturproteine zu den Kollagenen I bis V gehören. Durch die regelmäßige Wiederholung des Glycins an jeder dritten Position entsteht eine für Kollagen einzigartige Struktur, die aber nicht über die gesamte Kettenlänge verteilt sein muss. Die Primärstruktur der Polypeptidketten bildet eine linksgängige Helix.

Drei dieser Polypeptidketten können sich in Bereichen der Tripletts zu einer rechtsgängigen Tripelhelix zusammenlagern. Kollagenmoleküle bestehen aus tripelhelical zusammengelagerten Kollagenpeptidketten. Die Kollagenmoleküle können als Lösung vorliegen.

Unter fibrillärem Kollagen werden im Rahmen der Erfindung Kollagentripelhelices verstanden, die sich zu übergeordneten Strukturen mit einer definierten sich wiederholenden sogenannten Querstreifung von 63-70 nm zusammengelagert haben. Diese ist im Rasterkraftmikroskop, im Rasterelektronenmikroskop oder auch im Transmissionelektronenmikroskop darstellbar.

Unter Kollagenderivaten werden im Rahmen der Erfindung chemische und strukturelle Abkömmlinge des oben beschriebenen Kollagens verstanden, die bevorzugt durch technische Prozesse aus tierischem Gewebe oder aus durch gentechnische Veränderung gebildetem Kollagen aus Mikroorganismen oder auch aus chemisch synthetisierten Peptiden gewonnen werden und bei denen die tripelhelicale Struktur nicht oder nur noch teilweise vorhanden ist.

Unter einer Suspension werden im Rahmen der Erfindung eine nicht in Lösemittel vollständig lösliche Präparation aus Kollagen oder Kollagenderivaten verstanden.

Unter einer Lösung wird im Rahmen der Erfindung eine Präparation aus Kollagen oder Kollagenderivaten in Lösemittel, bevorzugt Wasser, organischen Säuren oder mit Additiven versetzten wässrigen Lösemitteln verstanden, die entweder vollständig filtrierbar ist oder das Filtrat einer Präparation darstellt.

Unter einer Separationsflüssigkeit wird im Rahmen der Erfindung eine nicht mit Wasser oder wässrigen Lösemitteln mischbare Flüssigkeit, z. B. Öle, verstanden.

Unter einer Vernetzungsflüssigkeit wird im Rahmen der Erfindung eine Flüssigkeit verstanden, die Kollagen oder Kollagenderivate vernetzende Komponenten enthält, z. B. chemische Vernetzer oder auch Enzyme.

Erfindungsgemäß wird zur Herstellung der Hohlkugeln aus Kollagen und mindestens einem Kollagenderivat in einem ersten Verfahrensschritt ein Behältnis bereitgestellt, in das eine Suspension oder Lösung mit Kollagen und mindestens einem Kollagenderivat, mindestens eine gegenüber der Suspension höher-viskose und niedriger-dichte Separationsflüssigkeit und mindestens eine gegenüber der Separationsflüssigkeit niedriger-viskose und niedriger-dichte Vernetzungsflüssigkeit angeordnet werden.

Um ein Vermischen der Suspension und der Vernetzungsflüssigkeit innerhalb des Behältnisses zu vermeiden, ist erfindungsgemäß vorgesehen, dass die Separationsflüssigkeit als phasentrennende Schicht zwischen der Suspension und der Vernetzungsflüssigkeit angeordnet wird, um ein Vermischen der Suspension und der Vernetzungsflüssigkeit zu vermeiden.

Zur Ausbildung gleichmäßiger und reproduzierbarer einzelner Hohlkugeln aus Kollagen und mindestens einem Kollagenderivat ist erfindungsgemäß vorgesehen, dass über mindestens eine Gaszuführeinrichtung ein Gas in die Suspension oder Lösung eingeleitet wird, wodurch aus den in die Suspension eingeleiteten Gasbläschen mit Gas gefüllte hohle Kugeln mit einer Kollagen-Kollagenderivat-Wandung ausgebildet und nachfolgend der Separationsflüssigkeit zugeführt werden.

In einer vorteilhaften Ausgestaltung des Verfahrens ist vorstellbar, dass eine steuerbare Gaszuführeinrichtung eingesetzt wird, mit der das zuführbare Gasvolumen der Gasmischung einstellbar ist und dadurch wesentlich auf die Größe und den Außendurchmesser der Hohlkugeln Einfluss genommen werden kann. Damit wird es möglich, dass kontrolliert Hohlkugeln aus Kollagen und mindestens einem Kollagenderivat mit frei variierbaren, aber definiert reproduzierbaren Außendurchmessern erhalten werden.

Vorstellbar ist auch, dass mehrere regelbare Gaszufuhreinrichtungen eingesetzt werden. Mit dem Einsatz mehrerer Gaszufuhreinrichtungen kann einerseits die Ausbeute an Hohlkugeln aus Kollagen und Kollagenderivat je Zeiteinheit erhöht werden. Andererseits besteht die Möglichkeit, die Gaszufuhreinrichtungen in variierender Höhe des Behältnisses vorzusehen. So ist vorstellbar, dass über eine erste Gaszufuhreinrichtung am Boden des Behältnisses eine Gasmischung der Suspension oder Lösung zugeführt wird, während eine zweite Gaszufuhreinrichtung in der seitlichen Behälterwandung eine weitere Gaszufuhr in die Suspension oder Lösung realisiert. Durch die unterschiedliche Steighöhe können Hohlkugeln mit unterschiedlichen Wandstärken ausgebildet werden, wodurch eine hohe Flexibilität des Verfahrens und der Vorrichtung hinsichtlich der Größe der Hohlkugeln ermöglicht wird.

Erfindungsgemäß durchdringen die in der Suspension oder Lösung gebildeten Hohlkugeln die Separationsflüssigkeit und werden nachfolgend einer Vernetzungsflüssigkeit zugeführt, wobei die Hohlkugeln in der Vernetzungsflüssigkeit stabilisiert werden, und nachfolgend die ausgebildeten Hohlkugeln aus der Vernetzungsflüssigkeit einzeln oder als Agglomerate entfernt werden, wobei danach weitere Behandlungen der Hohlkugeln, beispielsweise eine Nachvernetzung oder eine Besiedlungen oder Inkubation mit Zellen, durchgeführt werden können.

Es wurde festgestellt, dass die Viskosität insbesondere der Suspension oder Lösung ein wichtiger Parameter für die gezielte Ausbildung der Hohlkugeln aus Kollagen und mindestens einem Kollagenderivat ist. So hat sich als vorteilhaft herausgestellt, wenn die Viskosität der Suspension durch eine gezielte Temperierung in einem vorbestimmten Temperaturbereich eingestellt wird.

Vorteilhafterweise wird vorgeschlagen, dass mindestens die Suspension über eine zusätzliche thermische Quelle und/oder durch Zugabe mindestens eines Additives, beispielsweise Harnstoff, temperiert wird, wodurch einerseits die erforderliche Viskosität der Suspension, insbesondere des Kollagenderivates, eingestellt und andererseits eine Denaturierung des tripelhelicalen Kollagens verhindert wird. Zum Erreichen der gewünschten Viskosität wird vorteilhafterweise die Suspension oder Lösung auf eine Temperatur von 25°C bis 40°C eingestellt.

Im Ergebnis können eine definierte Auftriebsgeschwindigkeit der Gasbläschen innerhalb der Suspension oder Lösung eingestellt werden und damit kontrolliert auf die Ausbildung der Wandstärken der Hohlkugeln von vorteilhafterweise 5 µm bis ≤ 500 µm, bevorzugt 10 µm bis ≤ 200 µm, Einfluss genommen werden.

Eine derartige thermische Quelle kann beispielsweise innerhalb des Behältnisses im Bereich der eingefüllten Suspension vorgesehen sein. Möglich ist aber auch, dass das Behältnis von außen beheizt wird, beispielsweise, in dem das Behältnis in ein temperiertes Wasserbad eingebracht oder Wärme von einer externen thermischen Strahlungsquelle mittels elektromagnetischer Wellen übertragen wird.

Zur Ausbildung besonders elastischer und mechanisch belastbarer Hohlkugeln aus Kollagen und Kollagenderivat hat sich als vorteilhaft herausgestellt, wenn die verwendete Suspension oder Lösung einen Anteil von 1 Gew.-% bis 50 Gew.-% Kollagen und Kollagenderivat aufweist, wobei besonders vorteilhaft denaturierte Kollagentripelhelices, z. B. Gelatine oder Kollagenhydrolysat als Kollagenderivat Verwendung finden.

Zur Umsetzung einer sicheren Phasentrennung und zur Verhinderung einer Mischung von Suspension oder Lösung und Vernetzungsflüssigkeit wird vorgeschlagen, dass zwischen der Suspension oder Lösung und der Vernetzungsflüssigkeit eine Separationsflüssigkeit im Behältnis angeordnet wird, die vorteilhafterweise eine nichtwassermischbare Flüssigkeit ist und sowohl zur Suspension oder Lösung als auch zur Vernetzungsflüssigkeit eine unterschiedliche Dichte aufweist.

In einem nachgelagerten Verfahrensschritt kann vorteilhafterweise vorgesehen sein, dass die Vernetzungsflüssigkeit durch mindestens einen Reinigungsvorgang mit einem Lösemittel mindestens teilweise von der hergestellten Hohlkugel entfernt wird. Das mindestens teilweise Entfernen der Vernetzungsflüssigkeit von der Oberfläche der Hohlkugeln bietet den Vorteil, dass eine verbesserte Substratoberfläche bereitgestellt wird, auf die beispielsweise Gewebezellen oder Mikroorganismen angesiedelt werden können. Vorteilhafterweise wird dabei als Reinigungslösung eine alkoholische Lösung, besonders vorteilhaft z. B. Ethanol oder Isopropylalkohol, eingesetzt.

In einer weiteren vorteilhaften Ausgestaltung des Verfahren kann vorgesehen sein, dass nach dem Entfernen aus der Vernetzungsflüssigkeit die Hohlkugeln oder Agglomerate für einen zusätzlichen Nachvernetzungsschritt einer Nachvernetzungsflüssigkeit zugeführt werden, um eine weitere Verbesserung der multiaxialen Kompressionseigenschaften zu erreichen.

Vorteilhafterweise kann die Nachvernetzungsflüssigkeit mit vernetzenden Enzymen, bevorzugt Transglutaminase, und/oder Formaldehyd, bifunktionelle Aldehyde, Carbodiimide und/oder Isocyanate in wässriger Lösung eingesetzt werden.

Die aus Kollagen und mindestens einem Kollagenderivat hergestellten Hohlkugeln bieten die Möglichkeit, dass in das Innere der Hohlkugeln beispielsweise eine Zellsuspension, Gewebezellen und/oder Mikroorganismen und/oder eine Flüssigkeit mit bioaktiven Substanzen injiziert oder auf deren Wandung innen wie außen beispielsweise eine Gewebezellen und/oder Mikroorganismen inkubiert werden können, ohne dass dabei die Wandung der Hohlkugel beschädigt oder zerstört wird, da sich das Injektionsloch aufgrund der elastischen Eigenschaften selbständig wieder schließt.

Erfindungsgemäß wird auch eine Vorrichtung zur Durchführung des vorbeschriebenen Verfahrens bereitgestellt. Die Vorrichtung weist ein Behältnis mit mindestens einer Gaszuführeinrichtung auf, wobei im Behältnis eine Suspension oder Lösung mindestens aus Kollagen und mindestens einem Kollagenderivat, eine gegenüber der Suspension höher-viskose und niedriger-dichte Separationsflüssigkeit und eine gegenüber der Separationsflüssigkeit niedriger-viskose und niedriger-dichte Vernetzungsflüssigkeit vorhanden sind. Die Separationsflüssigkeit ist als phasentrennende Flüssigkeit zu verstehen, die die Suspension oder Lösung und die Vernetzungsflüssigkeit räumlich trennt.

Erfindungsgemäß ist die Gaszuführeinrichtung im Bereich der angeordneten Suspension oder Lösung realisiert.

Zudem weist die Vorrichtung im Bereich der Vernetzungsflüssigkeit eine Trennvorrichtung auf, mit der die hergestellten Hohlkugeln oder Agglomerate aus dem Behältnis entnommen werden können. Eine derartige Trennvorrichtung kann vorteilhafterweise ein trichterförmiges Bauteil, Überlaufanordnung, Abschöpfeinrichtung, Absaugvorrichtung und/oder eine pneumatische Vorrichtung sein.

In einer vorteilhaften Ausgestaltung der Vorrichtung kann zur Einstellung einer definierten Viskosität der Suspension oder Lösung vorgesehen sein, dass innerhalb und/oder außerhalb des Behältnisses mindestens im Bereich der Suspension oder Lösung eine thermische Quelle angeordnet ist.

Die Zuführung der Gasmischung in die Suspension aus Kollagen und mindestens einem Kollagenderivat kann beispielsweise über eine Öffnung im Boden des Behälters oder in der Seitenwand des Behälters erfolgen, was insbesondere Einfluss auf die mögliche Steighöhe der Gasbläschen bzw. Hohlkugeln innerhalb der Suspension oder Lösung und damit auf die Ausbildung der Wandstärke der Hohlkugeln hat.

In einer vorteilhaften Ausgestaltung der Vorrichtung kann es vorgesehen sein, dass zwei oder mehrere Gaszuführeinrichtungen vorhanden sind, die in unterschiedlicher Behälterhöhe die Gaszufuhr in die Suspension realisieren. Damit wird erreicht, dass in einem Verfahrensschritt zwei oder mehrere verschiedene Hohlkugeln mit unterschiedlichen Wandstärken hergestellt werden, wodurch die Variabilität und Effizienz der Vorrichtung und des Verfahrens verbessert werden.

Vorzugsweise lassen sich mit der erfindungsgemäßen Vorrichtung Hohlkugeln aus Kollagen und mindestens einem Kollagenderivat mit einem Außendurchmesser von 0,2 mm bis ≤ 1,5 cm bereitstellen, wobei die Schichtdicke der Wandung 5 µm bis ≤ 500 µm, bevorzugt 10 µm bis 200 µm, betragen kann.

Mit dem erfindungsgemäßen Verfahren und der erfindungsgemäßen Vorrichtung werden neuartige Hohlkugeln bereitgestellt, die eine multiaxial und elastisch verformbare Wandung aus Kollagen und mindestens einem Kollagenderivat aufweisen, wobei der ausgebildete Hohlraum mindestens mit einem Gas gefüllt ist. Die neuartigen Hohlkugeln weisen den wesentlichen Vorteil auf, dass durch die Kombination von Kollagen und mindestens einem Kollagenderivat die Wandung der Hohlkugel stabil ist, wodurch ein Zusammenfallen oder Platzen der Hohlkugel verhindert wird.

Kollagen ist das häufigste Eiweiß im menschlichen Körper, es ist biokompatibel und damit für in vitro-Modelle besser geeignet als andere synthetische Polymere. Die Kollagenstruktur ist entscheidend für die Zelle und weist besonders formstabile Eigenschaften auf. Zellen sind zudem in der Lage, sich mittels Zelladhäsionsmolekülen direkt an das Kollagen anzuhaften, sodass das eingesetzte Kollagen bei Hohlkugeln das passende Substrat für in vitro-Modelle bildet. Neben seiner Funktion als Strukturmolekül übt Kollagen wichtige Funktionen bei der Steuerung des Zellwachstums aus. Vorteilhafterweise kann fibrilliertes Kollagen verwendet werden, das gegenüber tripelhelicalem Kollagen verbesserte Stabilitätseigenschaften aufweist, den Hauptbestandteil der extrazellulären Matrix in allen mehrzelligen Lebewesen bildet und damit große Bedeutung für biomedizinische Zwecke hat.

Neben Kollagen, das tripelhelical und/oder vorteilhafterweise fibrillär sein kann, wird erfindungsgemäß auch mindestens ein Kollagenderivat als Zellsubstrat eingesetzt. Es kann durch Erhitzen von Kollagen erzeugt werden und stellt damit eine Abbauvariante der extrazellulären Matrix-Komponente dar. In einer bevorzugten Ausbildung der Erfindung wird als Kollagenderivat Gelatine verwendet. Im Gegensatz zum tripelhelicalen Kollagen ist Gelatine schaumstabilisierend, besitzt aber trotzdem die typischen Aminosäuresequenzen für die Erkennung und Anhaftung von Zellen.

Als tripelhelicales Kollagen kann vorteilhafterweise Kollagen vom Typ I, Typ II und/oder III oder auch andere Typen in Kombination mit mindestens einem Kollagenderivat in einer wässrigen Suspension oder Lösung verwendet werden. Die Komponenten sind sehr gut mischbar und zudem biokompatibel.

Die Wandung der gebildeten Hohlkugeln aus Kollagen und mindestens einem Kollagenderivat, auf der Zellen gesiedelt werden, besteht im Ergebnis aus zwei aneinandergrenzenden Zellschichten, getrennt durch eine Anhaftungsmatrix auf der Basis des Biomaterials Kollagen und seinen Derivaten. Die dünne elastische Wandung ermöglicht den Austausch von Sauerstoff, Nährstoffen und Abfallprodukten sowie eine multiaxiale Kompression entsprechend den mechanischen und physiologischen Eigenschaften beispielsweise von humanen Lungenbläschen.

Die technischen Vorteile und Wirkungen der Erfindung sind, dass
- ein sehr einfaches und kostengünstiges Verfahren sowie Vorrichtung zur Herstellung von Hohlkugeln aus Kollagen und Kollagenderivaten bereitgestellt wird,
- ein beispielsweise den humanen Lungenbläschen ähnliches ideales in vitro-Modell bereitgestellt wird,
- die Hohlkugeln aus Kollagen und Kollagenderivaten stabil gegenüber Scherstress, Kanülierung, Kulturmedien und Zellen sind,
- die Hohlkugeln aus Kollagen und Kollagenderivaten biokompatibel sind, und auf der Innenseite und auf der Außenseite mit unterschiedlichen Zellen besiedelbar sind,
- deren Gasraum mittels Injektion erreichbar ist,
- mit einfachen Mitteln auf die Parameter der physiologischen Eigenschaften der Hohlkugeln Einfluss genommen werden kann,
- die erzeugten Hohlkugeln in einer großen Breite technischer und medizinischer Einsatzgebiete Verwendung finden können,
- eine mechanische Stimulation in vitro an 3D-Geweben mit Lumen ermöglicht wird, und
- es erstmals möglich ist, hohle Kollagen-Kollagenderivat-Sphären in einem einfachen Prozess und ohne Zuhilfenahme synthetischer Polymere zu generieren.

Die Erfindung wird nachfolgend anhand eines Ausführungsbeispiels näher erläutert.

### Ausführungsbeispiel

Um aus einer wasserbasierten Suspension, enthaltend Kollagen und Typ A Gelatine mit einem Bloomwert 300, einzelne Hohlkugeln aus Kollagen und Kollagenderivaten herzustellen, wird sauer gelöstes Kollagen mit 0,04 Gew.-% (2 mg/ml Kollagen in 0,01 M HCl), Kollagenfasersuspension mit 1 Gew.-% und auf 40 °C erwärmte wässrige Gelatinelösung mit 26,7 Gew.-% gemischt und in ein ca. 35 cm langes, transparentes Röhrchen gefüllt, sodass eine 8 cm hohe Flüssigkeitssäule entsteht. Das Röhrchen wird über eine zusätzliche thermische Strahlungsquelle beheizt, sodass die Suspension auf eine Temperatur von 40°C eingestellt ist. Auf die ca. 5 cm hohe Kollagen-Gelatine-Suspension wird eine 5 cm hohe Schicht Rizinusöl als Separationsflüssigkeit appliziert. Dieses trennt die darauf angeordnete Vernetzungsflüssigkeit, die eine weniger dichte und niedrigviskose Ölphase (Paraffinöl mit Transglutaminase, 1 g Transglut. In 10 ml Öl) ist, von der Kollagen-Gelatine-Suspension.

Am unteren Ende des Röhrchens wird eine sterile sauerstoffhaltige Gasmischung in Form von Luft über einen Schlauch eingeleitet. Die Kollagenmischung wird im Wasserbad auf eine Temperatur von 40°C temperiert, um die Gelatine flüssig und niedrigviskos zu halten.

Zur Bildung der Hohlkugeln aus der Mischung aus Kollagen und Gelatine wird mit Hilfe einer Spritze ein definiertes Luftvolumen in den Schlauch und von da aus in die Suspension geleitet. Die eingeleiteten und entstehenden Luftbläschen steigen innerhalb der Suspension auf und werden dabei mit der Mischung aus Kollagen und Gelatine ummantelt. Anschließend gelangen sie durch die Separationsflüssigkeit in die Vernetzungsflüssigkeit, in der die Wandung der Hohlkugel stabilisiert wird. Nachdem die erzeugten Hohlkugeln aus Kollagen und Kollagenderivaten die Vernetzungsflüssigkeit passiert haben, gelangen sie in einen aufgesetzten Trichter, aus dem die resultierenden Hohlkugeln entnommen werden. Nunmehr stehen Hohlkugeln aus Kollagen und Gelatine mit einem einheitlichen Außendurchmesser von 300 µm bereit, die aufgrund des kontinuierlichen und konstanten eingeleiteten Luftvolumens im Wesentlichen identische Außendurchmesser aufweisen. Das überschüssige Öl der Separationsflüssigkeit und/oder Vernetzungsflüssigkeit wird von den abgenommenen Hohlkugeln durch Einlegen in Ethanol von der Oberfläche der Außenwandung der Hohlkugeln aus Kollagen und Kollagenderivaten entfernt. Im Ethanol befindet sich eine Vernetzungssubstanz, die die Wandung der Hohlkugel weiter stabilisiert. Nach mehrmaligem Waschen mit Kulturmedium werden die Hohlkugeln mit humanen Zellen beladen, um die Alveolarbarriere in Form einer Lungenepithelschicht, einer Schicht aus Endothelzellen und der Basalmembran auszubilden. Hierfür wird zunächst ein Gewebezelltyp in Form einer Suspension (Kulturmedium + Lungenepithelzellen) in das Innere der Hohlkugeln mittels Kanüle injiziert. Die befüllten Hohlkugeln werden anschließend 24 h in einem CO₂-lnkubator (37°C, 95% Luftfeuchte) kultiviert. Dabei haften die Zellen an der inneren Wandung an. Die Hohlkugeln lassen sich homogen besiedeln, wenn diese mindestens innerhalb der ersten 2 bis 4 Stunden regelmäßig gedreht werden.

Anschließend erfolgt eine Inkubation der Hohlkugeln aus Kollagen und Kollagenderivaten mit einer Zellsuspension aus primären Endothelzellen, die eine vollständige Besiedlung der Oberfläche zeigt. Hierfür werden die zell-beladenen Hohlkugeln in eine weitere Zellsuspension mit einem anderen Zelltyp getaucht und darin bis zu 24 h kultiviert (im CO₂-Inkubator bei 37 °C und 95% Luftfeuchte). Anschließend werden die mit Zellen besiedelten Hohlkugeln in ein Kulturgefäß mit frischem Medium überführt.

Beim zyklischen Anlegen von Druck auf einer in Flüssigkeit schwimmenden Hohlkugel können elastische Eigenschaften ähnlich den humanen Lungenbläschen mit einer wiederkehrenden Komprimierung und Entspannung der Geometrie der Hohlkugeln aus Kollagen und Kollagenderivaten beobachtet werden.

## Patentansprüche

1. Verfahren zur Herstellung von Hohlkugeln aus Kollagen und Kollagenderivaten, bei dem in ein Behältnis mindestens eine Suspension oder Lösung aus Kollagen und mindestens einem Kollagenderivat, mindestens eine gegenüber der Suspension höher-viskose und niedriger-dichte Separationsflüssigkeit und mindestens eine gegenüber der Separationsflüssigkeit niedriger-viskose und niedriger-dichte Vernetzungsflüssigkeit angeordnet werden, wobei die Separationsflüssigkeit als phasentrennende Schicht zwischen der Suspension und der Vernetzungsflüssigkeit angeordnet wird, nachfolgend über mindestens eine Gaszuführeinrichtung eine Gasmischung in die Suspension oder Lösung eingeleitet wird, wodurch aus den eingeleiteten Gasbläschen mit diesem Gas gefüllte Hohlkugeln mit einer Kollagen-Kollagenderivat-Wandung ausgebildet und der Separationsflüssigkeit zugeführt werden, nachfolgend die Hohlkugeln die Separationsflüssigkeit durchdringen und nachfolgend die Hohlkugeln der Vernetzungsflüssigkeit zugeführt werden, wobei die Hohlkugeln in der Vernetzungsflüssigkeit stabilisiert und vereinzelt werden, und nachfolgend die ausgebildeten Hohlkugeln aus der Vernetzungsflüssigkeit entfernt werden, wobei danach weitere Behandlungen der Hohlkugeln durchgeführt werden können.

2. Verfahren nach Anspruch 1, bei dem das Behältnis mindestens im Bereich der Suspension oder Lösung über eine zusätzliche thermische Quelle und/oder durch Zugabe mindestens eines Additives temperiert wird.

3. Verfahren nach Anspruch 2, bei dem als Additiv Harnstoff, Guanidinhydrochlorid und/oder andere hydrotropisch wirkende Additive zugegeben werden.

4. Verfahren nach Anspruch 2, bei dem zur Dichte- und Viskositätsregulierung wasserlösliche Oligo- oder Polymere, bevorzugt Kohlenhydrate und/oder Polysaccharide zugesetzt werden.

5. Verfahren nach Anspruch 1, bei dem eine steuerbare Gaszuführeinrichtung eingesetzt wird, mit der das zuführbare Gasvolumen der Gasmischung einstellbar und dadurch der Außendurchmesser der Hohlkugeln aus Kollagen und Kollagenderivaten variierbar ist.

6. Verfahren nach Anspruch 1, bei dem die Suspension oder Lösung mit einem Anteil von 1 Gew.-% bis 50 Gew.-% Kollagen und Kollagenderivat verwendet wird.

7. Verfahren nach Anspruch 1, bei dem als Separationsflüssigkeit eine nicht wassermischbare Flüssigkeit eingesetzt wird.

8. Verfahren nach Anspruch 1, bei dem die Separationsflüssigkeit durch mindestens einen Reinigungsvorgang mit einem Lösemittel mindestens teilweise von der hergestellten Hohlkugel entfernt wird, wobei besonders vorteilhaft als Lösemittel eine alkoholische Lösung, besonders vorteilhaft Ethanol, eingesetzt wird.

9. Verfahren nach Anspruch 1, bei dem in der hergestellten Hohlkugel und/oder auf dessen Wandung innen und/oder außen mindestens eine Zellsuspension, Gewebezellen und/oder Mikroorganismen inkubiert und/oder injiziert wird.

10. Verfahren nach Anspruch 1, bei dem die Hohlkugeln nach dem Entfernen aus der Vernetzungsflüssigkeit zur Nachvernetzung einer Nachvernetzungsflüssigkeit zugeführt werden, wobei besonders vorteilhaft in der Nachvernetzungsflüssigkeit vernetzende Enzyme, bevorzugt Transglutaminase, und/oder Formaldehyd, bifunktionelle Aldehyde, Carbodiimide und/oder Isocyanate in wässriger Lösung eingesetzt werden.

11. Verfahren nach Anspruch 1, bei dem das oder die Kollagenderivate durch Acrylate, Methacrylate und/oder Vinylverbindungen modifiziert werden.

12. Verfahren nach Anspruch 1, bei dem die Vernetzung und/oder die Nachvernetzung physikalisch durch elektromagnetische oder Teilchenstrahlung erfolgt.

13. Vorrichtung zur Durchführung des Verfahrens gemäß einem der vorhergehenden Ansprüche 1 bis 12, aufweisend ein Behältnis mit mindestens einer Gaszuführeinrichtung, wobei im Behältnis eine Suspension oder Lösung aus Kollagen und mindestens einem Kollagenderivat, eine gegenüber der Suspension höher-viskose und niedriger-dichte Separationsflüssigkeit und eine gegenüber der Separationsflüssigkeit niedriger- viskose und niedriger-dichte Vernetzungsflüssigkeit vorhanden sind, wobei die Gaszuführeinrichtung im Bereich der angeordneten Suspension realisiert ist, und wobei im Bereich der Vernetzungsflüssigkeit eine Trennvorrichtung angeordnet ist.

14. Vorrichtung nach Anspruch 13, bei der innerhalb und/oder außerhalb des Behältnisses mindestens im Bereich der Suspension oder Lösung eine thermische Quelle angeordnet ist.

15. Vorrichtung nach Anspruch 13, bei der die mindestens eine Gaszuführeinrichtung im Bereich des Behälterbodens oder in der Behälterseitenwand vorgesehen ist.

16. Vorrichtung nach Anspruch 13, bei der zwei oder mehrere Gaszuführeinrichtungen vorhanden sind, die in unterschiedlicher Behälterhöhe die Gaszufuhr in die Suspension ermöglichen.

17. Vorrichtung nach Anspruch 13, bei der die Trennvorrichtung ein trichterförmiges Bauteil, Überlaufanordnung, Abschöpfeinrichtung, Absaugvorrichtung und/oder eine pneumatische Vorrichtung ist.

18. Hohlkugel, die eine multiaxial und elastisch verformbare Wandung aus Kollagen und mindestens einem Kollagenderivat aufweist, wobei mindestens der ausgebildete Hohlraum mit mindestens einem Gas gefüllt ist, wobei besonders vorteilhaft das Kollagen einen oder mehrere unterschiedliche Kollagentypen enthält.

19. Hohlkugel nach Anspruch 18, bei der fibrilliertes Kollagen vorhanden ist.

20. Hohlkugel nach Anspruch 18, bei der als Kollagenderivat Gelatine vorhanden ist.

21. Hohlkugel nach Anspruch 18, bei der mindestens der Hohlraum eine Flüssigkeit, Mikroorganismen, Feststoffe und/oder Zellgewebe enthält.

22. Hohlkugel nach Anspruch 18, die einen Außendurchmesser von 0,2 mm bis ≤ 1,5 cm aufweist.

23. Hohlkugel nach Anspruch 18, bei der die Wandung eine Schichtdicke von 5 µm bis ≤ 500 µm, bevorzugt 10 µm bis 200 µm aufweist.
